# EUROPÄISCHE PATENTSCHRIFT

(11) **EP 1 360 499 B1**
(45) Veröffentlichungstag und Bekanntmachung des Hinweises auf die Patenterteilung: **28.06.2006**
(21) Anmeldenummer: 01984868.8
(22) Anmeldetag: 19.12.2001
(51) Int. Cl.: G01N 33/573, C07K 16/40

(54) **BESTIMMUNG DES ZYTOSOLISCHEN NADP-ABHÄNGIGEN MALATDECARBOXYLASE-ISOENZYMS**
DETERMINATION OF CYTOSOLIC NADP-DEPENDENT MALATE DEHYDROGENASE ISOENZYME
DETERMINATION DE L'ISOENZYME DE MALATE DEHYDROGENASE NADP DEPENDANTE CYTOSOLIQUE

(30) Priorität: 19.12.2000 DE 10063238; 19.12.2000 DE 10063237
(43) Veröffentlichungstag der Anmeldung: 12.11.2003
(73) Patentinhaber: ScheBo Biotech AG, 35394 Giessen (DE)
(72) Erfinder: SCHEEFERS, Hans, 35435 Wettenberg (DE); SCHEEFERS-BORCHEL, Ursula, 35435 Wettenberg (DE); MAZUREK, Sybille, 35440 Linden (DE); EIGENBRODT, Erich, 35440 Linden (DE)
(74) Vertreter: Dey, Michael
(86) Internationale Anmeldenummer: PCT/EP2001/015033
(87) Internationale Veröffentlichungsnummer: WO 2002/050546

(56) Entgegenhaltungen:
- EP-A- 0 384 130
- EP-A- 0 595 241
- US-A- 5 160 486
- WISE L S ET AL: "RAPID PURIFICATION AND RADIO IMMUNOASSAY OF CYTOSOLIC MALIC ENZYME" ANALYTICAL BIOCHEMISTRY, Bd. 140, Nr. 1, 1984, Seiten 256-263, XP001094779 ISSN: 0003-2697
- LOEBER G ET AL: "Characterization of cytosolic malic enzyme in human tumor cells." FEBS LETTERS. NETHERLANDS 16 MAY 1994, Bd. 344, Nr. 2-3, 16. Mai 1994 (1994-05-16), Seiten 181-186, XP002208660 ISSN: 0014-5793

## Beschreibung

Die vorliegende Erfindung betrifft Verfahren zum Nachweis eines malignen Geschehens in Menschen oder Tieren sowie einen Testkit zur Durchführung des erfindungsgemäßen Verfahrens und schließlich die Verwendung des Testkits zum selektiven Nachweis von cytosolischer NADP-abhängiger Malat-Decarboxylase insbesondere in menschlichen oder tierischen Körperflüssigkeiten oder in menschlichem oder tierischem Stuhl. Weiter betrifft die Erfindung monoklonale Antikörper, welche spezifisch die cytosolische NADP-abhängige Malat-Decarboxylase binden und mit keinem anderen Malat-Decarboxylase-Isoenzym kreuzreagieren.

NADP(H) ist ein wichtiges Coenzym, welches vor allem bei der Fett- und Cholesterin synthese für den Zellwandbau eine Rolle spielt. Gleichzeitig dient NADP(H) zur Entgiftung von Substanzen, die zur Chemotherapie von Tumoren eingesetzt werden. Die Zelle hat nur wenige Möglichkeiten, NADP(H) zu produzieren: die Glucose 6-Phosphat-Dehydrogenase-Reaktion, die 6-Phosphogluconat-Dehydrogenase-Reaktion, die Malat-Decarboxylase-Reaktion und die cytosolische lsocitrat-Dehydrogenase-Reaktion. Die beiden letzteren sind Enzyme der Glutaminolyse, die wie die Glycolyse in Tumoren generell verändert ist. Bei der Malat-Decarboxylase existieren die folgenden Isoenzyme: die cytosolische NADP-abhängige Malat-Decarboxylase (E.C. 1.1.1.40), die mitochondriale NAD-abhängige Malat-Decarboxylase (E.C. 1.1.1.39) und die mitochondriale NADP-abhängige Malat-Decarboxylase.Es wurde festgestellt, dass eine Überexpression der NADP-abhängigen Malat-Decarboxylase bei einer Vielzahl von Tumoren des Menschen zu beobachten ist (Loeber, G. et al., FEBS Lett. (1994), 344: 181-186; Moreadith, R. W. und Lehninger, A.L., J. Biol. Chem. (1984), 259: 6222-6227; Gerbracht et al., Carcinogenesis (1990), 1 1: 2111-2115; Chang et al., Eur. J. Biochem. (1991), 202: 681-688; Eigenbrodt et al., Critical Reviews in Oncogenesis (1992), 3 (1,2): 91-1 15; Mazurek et al., J. Biol. Chem. (1997), 272: 4941-4952; Mazurek et al., J. Cell. Physiol. (1999), 181: 136-146; Mazurek et al., in vivo (1999), 13: 467-478; Mazurek et al., Anticancer Res. (2000) in press. EP 595 241 A2 beschreibt ein Verfahren zur Inhibierung des Wachstums und der Vermehrung von Säugetier-Tumorzellen, dadurch gekennzeichnet, dass man die Tumorzellen darauf untersucht, ob sie Malatenzym und/oder welche Isoform von Matanenzym sie exprimieren und dass man das in den Zellen nachgewiesene Malatenzym spezifisch inhibiert, indem man seine Expression verhindert oder die Aktivität des Enzyms ganz oder teilweise inhibiert.

Insofern kann die NADP-abhängige Malat-Decarboxylase als eine Art Tumormarker (Parameter) betrachtet werden.

Bisher wurden diese Tumormarkereigenschaften jedoch nur insofern genutzt als die enzymatische Aktivität dieses Enzyms in Gewebehomogenaten geprüft wurde.

Tumormarker sind in Blut, Serum oder anderen Körperflüssigkeiten auftretende Makromoleküle oder Antigene, deren Konzentrationsänderung in gewisser Beziehung mit der Entstehung und dem Wachstum von malignen Tumoren eines Individuums stehen.

Der ideale Tumormarker sollte folgende Eigenschaften haben:
- hohe Spezifität, d.h. bei benignen Erkrankungen und gesunden Personen nicht nachweisbar sein;
- hohe Sensitivität, d.h. er kann in einem hohen Prozentsatz der Tumorpatienten nachgewiesen werden;
- gute Korrelation mit den Tumorstadien bzw. der Tumormasse;
- er sollte Auskunft über den Stoffwechsel des Tumors geben;
- seine Konzentration sollte gut mit der Tumormalignität korrelieren;
- Beziehung zur Prognose;
- verlässliche Vorhersagewerte.

Die Kriterien der 100 %igen Spezifität und der 100 %igen Sensitivität werden bis heute von keinem der bekannten Tumormarker erfüllt.

Es wäre wünschenswert, möglichst frühzeitig ein tumoröses Geschehen im Körper oder im Gastrointestinaltrakt, insbesondere in einer Wachstumsphase, in der der Tumor noch keinen Kontakt mit dem Gefäßsystem des Körpers aufgenommen hat (in der "Polyp-cancersequence", zeitlich vor der Infiltration der Submukosa) nachweisen zu können.

Beim Verdacht auf ein neoplastisches Geschehen im Gastrointestinaltrakt, besonders im Hinblick auf die sogenannte Adenom-Carcinom-Sequenz bei Polypen (Polyposis coli), wird nach dem Stand der Technik mittels verschiedener Bestimmungsmethoden versucht, occultes Blut im Stuhl nachzuweisen. Hierzu werden nicht-immunologische Tests (z.B. Pseudoperoxidase-Aktivität, Porphyrin-Nachweis) und immunologische Tests verwendet.

Beide Testprinzipien sind jedoch nicht sehr spezifisch, da sie durch eine Vielzahl von Einflussgrößen gestört werden können (falsch positiv/falsch negativ, z.B. durch Nichteinhalten dringend notwendiger Diätvorschriften von Seiten des Patienten und von einer Reihe von Arzneimitteln sowie durch überhöhte Vitamin-C-Gabe (z.B. in Gemüse, Fruchtsäften etc.) (Thomas, L., Labor und Diagnose, 5. Auflage, 1998).

Ein positiver Test auf occultes Blut im Stuhl muss so lange abgeklärt werden, bis die Blutungsquelle lokalisiert ist bzw. die Ursache der Blutung gefunden wurde. Der klinische Befund rechtfertigt in jedem Fall eine zügig durchzuführende weitere Diagnostik, z.B. durch Endoskopie, Sonographie, Röntgen. Das Vorliegen von Blut im Stuhl ist aber nicht immer auf ein tumoröses Geschehen zurückzuführen und andersherum bedeutet das Fehlen von Blut im Stuhl auch nicht sicher, dass kein Tumor im Darm vorhanden ist.

Die vorliegende Erfindung hat zum Ziel, den weiterhin bestehenden Bedarf an einem spezifischen, leicht durchzuführenden Verfahren zu befriedigen, sodass ein neoplastisches Geschehen im Körper bzw. im Hinblick auf die Problematik der sogenannten Adenom-Carcinom-Sequenz bei Polypen (Polyposis coli) im Gastrointestinaltrakt, besonders frühzeitig und zweifelsfrei nachgewiesen werden kann.

Diese Aufgabe wird gelöst durch ein Verfahren zum Nachweis eines malignen Geschehens in Menschen oder Tieren, insbesondere im Körper oderim Gastrointestinaltrakt, bei dem man in einer Körperflüssigkeitsprobe oder/und in einer Stuhlprobe das Glutaminolyse-Isoenzym cytosolische NADP-abhängige Malat-Decarboxylase oder Teilstücke derselben qualitativ oder/und quantitativ bestimmt.

Es wurde im Rahmen der vorliegenden Erfindung festgestellt, daß sich der Gehalt an cytosolischer NADP-abhängiger Malat-Decarboxylase in Körperflüssigkeiten oder im Stuhl von Tumorpatienten bestimmen lässt, wobei der Gehalt direkt proportional zum Grad des malignen Geschehens ist.

Dies ist umso überraschender, weil umfangreiche Untersuchungen bezüglich anderer spezifischer Proteine in Körperflüssigkeiten bzw. im Stuhl keine Hinweise auf ihre mögliche Verwendung als Tumormarker gaben.

Sowohl die Struktur als auch die physiko-chemischen Eigenschaften der cytosolischen NADP-abhängigen Malat-Decarboxylase werden offensichtlich nicht infolge der erheblichen proteolytischen Aktivität und den extremen physiologischen Gegebenheiten in den Körperflüssigkeiten oder im Gastrointestinaltrakt z.B. pH-Wert, sauer im Magen, alkalisch im Darm, nachhaltig beeinträchtigt. Dies gilt auch für den Nachweis der cytosolischen NADP-abhängigen Malat-Decarboxylase mittels immunologischer Methoden.

Im Rahmen der vorliegenden Erfindung konnte festgestellt und gezeigt werden, dass sich trotz der oben beschrieben Proteindenaturierung und Proteinverdauung ein spezifischer qualitativer und vorzugsweise sogar quantitativer Nachweis von cytosolischer NADP-abhängiger Malat-Decarboxylase in Körperflüssigkeiten bzw. im Stuhl von Tumorpatienten führen lässt.

Überraschenderweise wurde dabei auch festgestellt, dass die cytosolische NADP-abhängige Malat-Decarboxylase quantitativ auch bei intensiv homogenisierten, länger gelagerten Proben (zum Beispiel beim Probenversand) nachweisbar bleibt. Auch bei starker Verdünnung des Stuhles kann eine deutliche Reaktion erhalten werden. Hierbei wurde überraschenderweise festgestellt, dass der Nachweis auch ohne vorherige Extraktion in der Stuhlprobe selektiv erfolgt. Vorzugsweise ist jedoch ein Extraktionsverfahren unter vorzugsweiser Verwendung eines speziellen Detergens (CHAPS) zu verwenden, welches beispielhaft in Beispiel 2 beschrieben ist.

Geeignete Körperflüssigkeiten, die zur Durchführung des erfindungsgemäßen Verfahrens herangezogen werden können, umfassen beispielsweise Plasma, Serum, cerebrospinales Fluid, Ejakulat, Speichel oder/und Urin. Besonders bevorzugt wird das erfindungsgemäße Verfahren unter Verwendung von Plasma oder/und Serum durchgeführt.

Weiterhin kann mit dem erfindungsgemäßen Verfahren auch ein malgines Tumorwachstum im Gastrointestinaltrakt, insbesondere in der Speiseröhre, im Magen, in der Bauchspeicheldrüse, im Dünndarm oder im Dickdarm diagnostiziert werden. Der Gastrointestinaltrakt ist biologisch gesehen ein außerhalb des Körpers befindlicher Bereich. Bei einem Nachweis des Glutaminolyse-Isoenzyms cytosolische NADP-abhängige Malat-Decarboxylase im Stuhl wird somit unmittelbar ein Tumor im Gastrointestinaltrakt angezeigt und somit eine Lokalisierung des Tumors im Gastrointestinaltrakt möglich.

Ein weiterer Vorteil des erfindungsgemäßen Verfahrens besteht darin, dass Tumore im Körper bzw. im Gastrointestinaltrakt in einem nicht-invasiven Verfahren auf einfache Weise durch Bestimmung eines Tumormarkerproteins nachgewiesen werden können.

Beim erfindungsgemäßen Vefahren wird die Bestimmung der cytosolischen NADP-abhängigen Malat-Decarboxylase vorzugsweise mittels eines Immunoassays unter Verwendung mindestens eines Antikörpers durchgeführt, der spezifisch die cytosolische NADP-abhängige Malat-Decarboxylase oder Teilstücke derselben erkennt und mit keinem anderen Malat-Decarboxylase-Isoenzym oder Teilen derselben kreuzreagiert. In einer besonders bevorzugten Ausführungsform der Erfindung verwendet man als einen solchen spezifischen Antikörper einen monoklonalen Antikörper, insbesondere einen Antikörper, der aus der Hybridoma-Zelllinie Klon I erhältlich ist, oder einen Antikörper mit gleicher Spezifität und Selektivität.

Erfindungsgemäß einsetzbare Antikörper können gemäß im Stand der Technik bekannten Verfahren hergestellt werden. Dem Fachmann sind Verfahren zur Herstellung von spezifischen monoklonalen Antikörpern gut bekannt. Beispielsweise wird hierzu das Antigen, im vorliegenden Fall also die cytosolische NADP-abhängige Malat-Decarboxylase oder Teilstücke derselben in in üblicher Weise gereinigter Form zur Erzeugung von Antikörpern verwendet. Prinzipiell kann hierzu das Verfahren, welches erstmals von Köhler und Milstein beschrieben wurde, angewandt werden, wobei dem Fachmann auch Abwandlungen und Weiterentwicklungen dieser Verfahren bekannt sind. Die Herstellung ist solange nicht kritisch, wie spezifische Antikörper erhalten werden, was durch Selektion festgestellt werden kann. Die Selektion erfolgt auf Antikörper, welche spezifisch die cytosolische NADP-abhängige Malat-Decarboxylase oder Teilstücke derselben, jedoch nicht eines der anderen Isoenzyme der Malat-Decarboxylase binden.

Ein erfindungsgemäß bevorzugter Immunoassay wird in der Weise durchgeführt, dass man
a) die Probe mit mindestens zwei verschiedenen Rezeptoren in Kontakt bringt, von denen der erste Rezeptor R1 in fester Phase vorliegt und mit der cytosolischen NADP-abhängigen Malat-Decarboxylase bindefähig ist und der zweite Rezeptor R2 in flüssiger Phase vorliegt und ebenfalls mit der cytosolischen NADP-abhängigen Malat-Decarboxylase bindefähig ist, und wobei Rezeptor R2 eine Markierung trägt oder die Bindung an ein detektierbares Molekül vermittelt,
b) die feste von der flüssigen Phase trennt,
c) die Markierung oder das detektierbare Molekül in der festen Phase bestimmt und entsprechend die Menge an in der Probe vorhandener cytosolischer NADP-abhängiger Malat-Decarboxylase quantifiziert, wobei man als mindestens einen der Rezeptoren R1 oder R2 einen Antikörper verwendet, der spezifisch mit der cytosolischen NADP-abhängigen Malat-Decarboxylase bindefähig ist und kein anderes Malat-Decarboxylase-Isoenzymen bindet.

Bei Durchführung eines erfindungsgemäßen Immunoassays kann spezifisch der Gehalt an cytosolischer NADP-abhängiger Malat-Decarboxylase festgestellt werden, welche als Tumormarker angesehen werden kann. Durch die erfindungsgemäße Verwendung mindestens eines Antikörpers, der mit keinem anderen Malat-Decarboxylase-Isoenzym kreuzreagiert, kann in besonders spezifischer Weise dasjenige Malatdecarboxylase-Isoenzym nachgewiesen werden, welches sich als Tumormarker verwenden lässt. Die anderen Malat-Decarboxylase-Isoenzyme, welche oben beschrieben sind, sind keine verlässlichen Tumormarker, wohingegen überraschenderweise die cytosolische NADP-abhängige Malat-Decarboxylase äußerst spezifisch und selektiv bei Tumorpatienten im Stuhl nachgewiesen werden kann.

In einer besonders bevorzugten Ausführungsform der Erfindung verwendet man den spezifisch mit der cytosolischen NADP-abhängigen Malat-Decarboxylase bindefähigen und nicht mit anderen Malat-Decarboxylase-Isoenzymen kreuzreagierenden Antikörper als Rezeptor R1, d.h. als den an die feste Phase gebundenen Rezeptor.

Der zweite Rezeptor R2 ist dann weniger kritisch, sodass in diesem Fall auch ein Rezeptor verwendet werden kann, der cytosolische NADP-abhängige Malat-Decarboxylase bindet, der jedoch zumindest in gewisser Menge Kreuzreaktionen mit anderen Isoenzymen zeigt.

Dadurch, dass allerdings an der festen Phase lediglich spezifisch die cytosolische NADP-abhängige Malat-Decarboxylase gebunden vorliegt, kann sich bei der Immunreaktion an der festen Phase nur dann ein über die Markierung nachweisbarer Immunkomplex bilden, wenn tatsächlich die cytosolische NADP-abhängige Malat-Decarboxylase in der Probe vorhanden ist.

Um den Test ganz besonders spezifisch zu machen und Hintergrund und unspezifischen Background weitestgehend auszuschließen, verwendet man in einer besonders bevorzugten Ausführungsform auch als Rezeptor R2 einen ebenfalls spezifisch mit der cytosolischen NADP-abhängigen Malat-Decarboxylase bindefähigen Antikörper, der nicht mit anderen Isoenzymen kreuzreagiert.

Es ist aber auch möglich als Rezeptor R1 einen weniger spezifischen Antikörper einzusetzen und als Rezeptor R2 einen Antikörper zu verwenden, der spezifisch mit der cytosolischen NADP-abhängigen Malat-Decarboxylase bindefähig ist und mit keinem anderen Malat-Decarboxylase-Isoenyzm kreuzreagiert, wobei ein detektierbares Signal ebenfalls nur bei Vorhandensein von cytosolischer NADP-abhängiger Malat-Decarboxylase entsteht.

Wie oben bereits erwähnt, können jedoch auch zwei spezifisch nur mit cytosolischer NADP-abhängiger Malat-Decarboxylase bindefähige Antikörper eingesetzt werden, um eine möglichst hohe Spezifität zu erreichen.

In einer bevorzugten Ausführungsform des erfindungsgemäßen Verfahrens verwendet man als Rezeptor R1 einen spezifisch mit cytosolischer NADP-abhängiger Malat-Decarboxylase bindefähigen und zwischen anderen Malat-Decarboxylase-Isoenzymen diskriminierenden Antikörper.

Weiterhin ist es bevorzugt, als Rezeptor R2 einen Antikörper zu verwenden, der selbst eine Markierung trägt. Damit ist ein direkter Nachweis noch schneller möglich als in dem Fall, in dem ein detektierbares Molekül an den Rezeptor R2 gebunden ist.

In einer weiteren bevorzugten Ausführungsform der Erfindung verwendet man als Rezeptor R2 einen Enzym-gebundenen Antikörper und führt das Verfahren in Form eines ELISA (Enzyme Linked Immuno Sorbent Assay) durch. Das Enzym ist somit das detektierbare Molekül, welches über den Immunkomplexrezeptor [R2 - cytosolische NADP-abhängige Malat-Decarboxylase - Rezeptor R1] an die feste Phase gebunden vorliegt.

Neben der Durchführung des erfindungsgemäßen Verfahrens in Form eines Immunassays und insbesondere eines ELISA ist allerdings auch der Nachweis der cytosolischen NADP-abhängigen Malat-Decarboxylase in einer Körperflüssigkeitsprobe oder in einer Stuhlprobe mit Hilfe anderer Nachweisverfahren möglich, wobei z.B. der Nachweis mit Hilfe von Massenspektroskopie, insbesondere von MALDI-TOF. (Matrix unterstütze Laserdesorption/lonisations-Flugzeitmassenspektrometrie), geeignet erscheint.

Neben der Bestimmung der cytosolischen NADP-abhängigen Malat-Decarboxylase mittels Immunoassay und insbesondere ELISA oder anderen Sandwich-Assays kann die Analytkonzentration prinzipiell auch mittels Biosensoren bestimmt werden, wie z.B. amperometrische Sensoren, potentiometrische, ionenselektive potentiometrische oder fotometrische Sensoren, oder auch mittels Halbleiterelektroden, wie Feldeffekttransistoren (FET), chemosensitive Feldeffekttransistoren (CHEMFET), suspendedgate-Feldeffekt-Transistoren (SGFET) oder ionensensitive Feldeffekttransistoren.

Derartige Biosensoren sind zusammenfassend in E.A. H. Hall und G. Hummel in "Biosensoren", Springer Verlag Heidelberg, Deutschland, 1995 beschrieben. Weitere Entwicklungen von ionen-sensitiven Feldeffekttransistoren (ISFET) oder optischen Detektoren sind u.a. von F. Aberl und H. Wolf in "Aktuelle Trends in der Immunsensorik", Labor 2000, S. 70-96 (1993) beschrieben. Ebenfalls geeignet ist das erfindungsgemäße Verfahren für die Durchführung mittels piezoelektrischen Schwingquarzen und Oberflächenwellenelementen, welche als Mikrowaagen verwendet werden können. Dabei wird der primäre Antikörper (der sog. Catcher) auf einem piezoelektrischen Substrat immobilisiert und nach Bindung mit der zu analysierenden cytosolischen NADP-abhängigen Malat-Decarboxylase gemessen. Derartige Sensoren sind beispielsweise von A. Leidl et al. In "Proceedings of the Second International Symposium on Minaturized Total Analyses Systems *µ*TAS", Basel 1996, beschrieben. Quarzkristallmikrowaagen, wie sie von C. Köslinger et al., Fresenius J. Anal. Chem (1994), 349: 349-354, beschrieben sind, haben sich als besonders geeignet erwiesen.

Signifikante Verbesserungen der Nachweismöglichkeiten durch Immunoassays können hinsichtlich Sensitivität, Dynamik, Assaykinetik und Formatflexibilität auch durch die Verwendung von Elektrochemilumineszenz erreicht werden. Die Elektrochemilumineszenz ist der Vorgang, bei dem eine Lichterzeugung dann auftritt, wenn eine niedrige Spannung an eine Elektrode angelegt wird, welche eine cyclische Redoxreaktion eines Rutheniummetallion triggert (Bruno, G. (1997) Rec. Rp S. 175-179; Williams R. (1996), Amer. Biotech., p. 26).

Neben dem erfindungsgemäßen Verfahren ist ein weiterer Gegenstand der vorliegenden Erfindung ein Testkit zum Nachweis oder/und zur Diagnose eines malignen Tumorgeschehens in Menschen oder Tieren, welcher mindestens einen mit der cytosolischen NADP-abhängigen Malat-Decarboxylase spezifisch bindenden Rezeptor enthält, der mit keinem anderen Malat-Decarboxylase-Isoenzym kreuzreagiert, sowie gegebenenfalls weitere für die Durchführung eines Immunoassays notwendige Reagenzien, wobei der Testkit in Form eines Teststreifens (2) ausgebildet ist, der eine Auftragzone (4) für die Probe aufweist und weitere Abschnitte aufweist, welche so ausgebildet sind, dass eine im Probeauftragbereich (4) aufgetragene Flüssigkeit enthaltende Probe bis zu einer Detektionszone (8) durchwandert, in der das Testergebnis abgelesen werden kann.

Der Testkit ist insbesondere zum Nachweis oder/und zur Diagnose eines malignen Tumorgeschehens im Körper oder/und im Gastrointestinaltrakt von Menschen und Tieren und bevorzugt im Darm von Menschen oder Tieren vorgesehen.

Der Testkit ist insbesondere für die Durchführung des erfindungsgemäßen Verfahrens ausgestaltet.

Der erfindungsgemäße Testkit enthält dann vorzugsweise zusätzlich mindestens einen weiteren Rezeptor, der ebenfalls an die cytosolische NADP-abhängige Malat-Decarboxylase bindet und eine Markierung trägt oder die Bindung an ein detektierbares Molekül vermittelt.

Einer der beiden Rezeptoren vermittelt vorzugsweise die Bindung an eine feste Phase, sodass eine Trennung von flüssiger Phase, enthaltend die Stuhlprobe, und fester Phase mit daran über einen der Rezeptoren gebundene cytosolische NADP-abhängige Malat-Decarboxylase ermöglicht wird. Diese Festphase ist in einer bevorzugten Ausgestaltung der Erfindung ebenfalls Bestandteil des Testkits. Die zu untersuchende Probe wird dann mit dem vorzugsweise gelösten Protein in Kontakt gebracht und in einem geeigneten Puffersystem mittels der Antikörper an die Festphase gebunden. Nach Waschen des so erhaltenen immobilisierten Antikörper-cytosolische NADP-abhängige Malat-Decarboxylase-Komplexes wird dann ein weiterer markierter, z.B. mit Biotin versehener zweiter Antikörper zugesetzt, der an ein anderes Epitop der cytosolischen NADP-abhängigen Malat-Decarboxylase bindet. Dann wird die Markierung bestimmt, z.B. mit Hilfe von Streptavidin-Peroxidase. Die Menge an gebundenem Marker ist direkt proportional der Menge an cytosolischer NADP-abhängiger Malat-Decarboxylase in der Probe. Zweckmäßigerweise enthält der Testkit Referenzmaterial bekannten Gehalts an cytosolischer NADP-abhängiger Malat-Decarboxylase zur quantitativen Bestimmung.

Neben der bevorzugten Ausgestaltung als Festphasen-Sandwich-Assay sind allerdings auch andere Nachweismethoden und Immunoassays geeignet, mit deren Hilfe die cytosolische NADP-abhängige Malat-Decarboxylase spezifisch nachgewiesen werden kann, sowie Testkits, die die dafür notwendigen Reagenzien enthalten, insbesondere den spezifisch bindefähigen Antikörper.

Selbstverständlich können auch alle anderen Arten von immunologischen Nachweisverfahren, welche mit Hilfe von Antikörpern durchgeführt werden, erfindungsgemäß eingesetzt werden. Gerade für die tatsächliche Formulierung als Testkit sind dem Fachmann viele Alternativen bekannt, die allesamt prinzipiell für die Durchführung des erfindungsgemäßen Verfahrens geeignet sind. So kann auch beispielsweise ein Test in Form eines Teststreifens konzipiert werden, auf dem in unterschiedlichen Zonen des Teststreifens die benötigten Antikörper entweder in löslicher Form oder Festphasen fixiert angeordnet sind. Die Probe bzw. der Flüssigkeitsanteil der Probe oder ein Extrakt können dann den Teststreifen durchwandern und an der Detektionsstelle ein Signal liefern, wenn cytosolische NADP-abhängige Malat-Decarboxyalse in der Probe vorhanden ist. Die genaue Anordnung der einzelnen Komponenten auf dem Teststreifen ist abhängig vom angewandten immunologischen Verfahren und vom Fachmann leicht realisierbar.

Im Rahmen der vorliegenden Erfindung soll der Ausdruck "Rezeptor" bzw. "Antikörper" auch solche Teile oder Fragmente von Rezeptoren bzw. Antikörpern umfassen, welche die notwendige Bindung an cytosolische NADP-abhängige Malat-Decarboxylase noch vermitteln. Es ist hierbei auch möglich, anstelle eines einzelnen Antikörpers ein Konjugat, beispielsweise aus zwei Antikörpern einzusetzen. Insbesondere kann man sich vorstellen als Rezeptor R2 einen mit cytosolischer NADP-abhängiger Malat-Decarboxylase bindefähigen Antikörper zu verwenden und den Nachweis über einen weiteren markierten Antikörper zu führen, welcher gerichtet ist gegen den Fc-Teil des Rezeptors R2 und eine Markierung trägt oder wiederum an ein detektierbares Molekül gekoppelt ist. Diese Konjugatbildung aus zwei Antikörpern soll im Rahmen der vorliegenden Erfindung mitumfasst sein, wenn R2 so definiert ist, dass er die Bindung an ein detektierbares Molekül vermittelt. Analoges gilt für die Bindung des Rezeptors R1 an die feste Phase. Auch diese Bindung kann über Festphasen-gekoppelte Antikörper erfolgen, an welche der Fc-Teil des Rezeptors R1 bindet.

Viele weitere Ausgestaltungen der Erfindung sind denkbar und für den Fachmann ohne weiteres durchführbar. Diese Ausgestaltungen sind daher im Rahmen der vorliegenden Erfindung mitumfasst, solange sie den Nachweis der cytosolischen NADP-abhängigen Malat-Decarboxylase in einer Körperflüssigkeitsprobe oder/und in einer Stuhlprobe ermöglichen.

Im Rahmen der vorliegenden Erfindung ist es bevorzugt, dass der Testkit als Rezeptoren R1 oder/und R2 Antikörper oder spezifisch bindefähige Antikörperteile enthält. Es ist weiterhin bevorzugt, dass Rezeptor R1 an eine feste Phase gekoppelt vorliegt und besonders bevorzugt Rezeptor R1 ein monoklonaler Antikörper ist, der spezifisch an cytosolische NADP-abhängige Malat-Decarboxylase bindet und nicht mit anderen Malat-Decarboxylase-Isoenzymen kreuzreagiert.

Im Rahmen der vorliegenden Erfindung ist der Rezeptor R2 bevorzugt ein löslicher, markierter Antikörper oder ein löslicher Antikörper, der an ein Enzym gebunden ist, welches wiederum über eine Nachweisreaktion bestimmbar ist. Es ist besonders bevorzugt, dass der Testkit so ausgestaltet ist, dass er weitere für einen Sandwich-, ELISA-, Schwingquarz-, Mikrowaage- oder Elektrochemolumineszenztest notwendige Reagenzien oder/und Vorrichtungen enthält.

Bei Durchführung des erfindungsgemäßen Verfahrens in Form eines Sandwich- bzw. ELISA-Immunoassays ist es im Rahmen der vorliegenden Erfindung wiederum bevorzugt, dass der Testkit in Form eines Teststreifens ausgebildet ist, in dem die Probenflüssigkeit verschiedene Bereiche durchläuft, in denen sich die Rezeptoren R1 und R2 in löslicher Form befinden. Dabei ist der Testkit in Form eines Teststreifens (2) ausgebildet ist, der eine Auftragzone (4) für die Probe aufweist und weitere Abschnitte aufweist, welche so ausgebildet sind, dass eine im Probenauftragbereich (4) aufgetragene flüssige Probe bis zu einer Detektionszone (8) durchwandert, in der das Testergebnis abgelesen werden kann. Die verschiedenen Bereiche müssen also z.B. durch Kapillarwirkung die Wanderung der Probe unterstützen.

Dabei ist es wiederum bevorzugt, dass auf die Auftragzone ein Bereich (6) folgt, in dem ein löslicher markierter Antikörper vorhanden ist, der Rezeptor R2 entspricht. In einer darauf folgenden Detektionszone (8) liegt Rezeptor R1 vorzugsweise bereits in immobilisierter Form vor. Allerdings kann auch der Rezeptor 1 sowohl in einer Zone (6') vor als auch in einer Zone nach dem Bereich (6) vorliegen und dort in löslicher Form vorhanden sein. Es muss dann eine feste Phase (10) auf dem Testkit vorhanden sein, in welcher der Rezeptor R1 an die feste Phase bindet und dadurch ein Komplex aus Rezeptor R1, cytosolischer NADP-abhängiger Malat-Decarboxylase und Rezeptor R2 mit der Markierung bzw. den detektierbaren Molekülen, an die Festphase gebunden wird. Die Anwesenheit der cytosolischen NADP-abhängigen Malat-Decarboxylase kann dann wiederum über die Markierung festgestellt werden, wobei diese Markierung so ausgestaltet sein kann, dass sie ohne weitere Zugaben von Substanzen erkennbar ist, wie z.B. eine Goldmarkierung oder eine Fluoreszenzmarkierung, oder aber auch so ausgestaltet sein kann, dass eine Zugabe weiterer Reagenzien die Bestimmung ermöglicht. Beispielsweise kann ein Enzymsubstrat in einem zweiten Schritt auf den Teststreifen aufgegeben werden, oder aber in einem separaten Bereich des Teststreifens anwesend sein, den die Probenflüssigkeit einschließlich Rezeptor R2 durchwandert.

In einer bevorzugten Ausführungsform der Erfindung ist der Teststreifen so angeordnet, dass zwischen der Auftragzone (2) und den weiteren Bereichen des Teststreifens ein Filter (12) angeordnet ist, der unlösliche Bestandteile der Probe zurückhält.

Um eine zügige Durchwanderung des Teststreifens zu gewährleisten, kann hinter den Detektionsbereich (8) ein flüssigkeitsaufsaugendes Medium (14) aufgebracht sein, welches im Wesentlichen die gesamte Flüssigkeitsmenge der aufgetragenen Probe aufsaugen kann.

Die Anwesenheit eines solchen flüssigkeitsaufsaugenden Mediums verstärkt die Kapillarkräfte, welche die Durchwanderung des Teststreifens durch die Probenflüssigkeit vorantreiben.

Bevorzugt ist der lösliche Rezeptor R2 ein markierter Antikörper.

Ein weiterer Gegenstand der vorliegenden Erfindung ist die Verwendung des erfindungsgemäßen Testkits zum qualitativen oder/und quantitativen selektiven Nachweis von cytosolischer NADP-abhängiger Malat-Decarboxylase in menschlichen oder tierischen Körperflüssigkeiten oder in menschlichem oder tierischem Stuhl.

In der vorliegenden Erfindung kann ein monoklonaler Antikörper eingesetzt werden, welcher spezifisch die cytosolische NADP-abhängige Malat-Decarboxylase bindet und mit keinem anderen Malat-Decarboxylase-Isoenzym kreuzreagiert.

Die Antikörper sind mit Hilfe von an sich bekannten Methoden erhältlich. Hierbei wird zuerst die cytosolische NADP-abhängige Malat-Decarboxylase, z.B. aus Dickdarmtumoren, menschlicher Leber und Brusttumoren, isoliert. Die Reinigung des Isoenzyms besteht aus vier Schritten: einer Ammonsulfatfällung, QAE Sephadex A-50-, 2'5'-ADP-Sepharose 4B- und einer Sephacryl-S300-Chromatographie, wie bei Oluyinka O. Irukera beschrieben (Dissertation an der Justus-Liebig-Universität in Gießen, Juli 1998). Die gereinigte Fraktion ergab nach SDS-Gel-Elektrophorese eine Bande mit einem Molekulargewicht von 60.000 Da.

Danach wird ein Versuchstier mit der so erhaltenen cytosolischen NADP-abhängigen Malat-Decarboxylase bzw. mit Bruckstücken derselben, welche die entsprechenden Epitope aufweisen, immunisiert und die gebildeten Antikörper isoliert. Derartige Bruchstücke, welche zur Immunisierung verwendet werden, können z.B. aus einer Protease-Verdauung der gereinigten cytosolischen NADP-abhängigen Malat-Decarboxylase stammen oder aus synthetischen Teilpeptiden derselben bestehen. Die Herstellung solcher Teilpeptide ist dem Fachmann an sich bekannt. Es können dabei aus der Gesamtsequenz z.B. mit Hilfe von Computerprogrammen Teilsequenzen ausgewählt werden, welche entsprechende Epitope enthalten. Diese Sequenzen werden dann auf ihre Verwendbarkeit zur Herstellung spezifischer Antikörper getestet.

Vorzugsweise werden dabei monoklonale Antikörper nach der Methode von Köhler, Milstein (Nature 256, 495-497 (1975) erzeugt. Dabei werden beispielsweise BALB/c-Mäuse mit isolierter cytosolischer NADP-abhängiger Malat-Decarboxylase immunisiert und die Milzzellen dieser Tiere mit einer Myelomazelfinie, beispielsweise PA I, fusioniert. Die in den Ascites sezernierten Antikörper werden, beispielsweise im ELISA oder RIA (Radio Immunoassay), auf ihre Spezifität getestet und isoliert. Üblicherweise gehören die so erhaltenen Antikörper zur Klasse IgG1.

In der vorliegenden Erfindung können auch Aptamere eingesetzt werden, welche spezifisch an die cytosalische NADP-abhängige Malat-Decarboxylase binden und mit keinem anderen Malat-Decarboxylase-Isoenzym kreuzreagieren.

Aptamere sind Oligonukleotidsequenzen, welche spezifische Bindeeigenschaften aufweisen. Die erfindungsgemäßen Aptamere können beispielsweise nach den in US 5,270,163 oder in Sumedha, Clin. Chem. 45 (1999) 1628-1650 beschriebenen Methoden hergestellt bzw. identifiziert werden.

Die Erfindung wird durch die folgenden Figuren und Beispiele weiter erläutert.

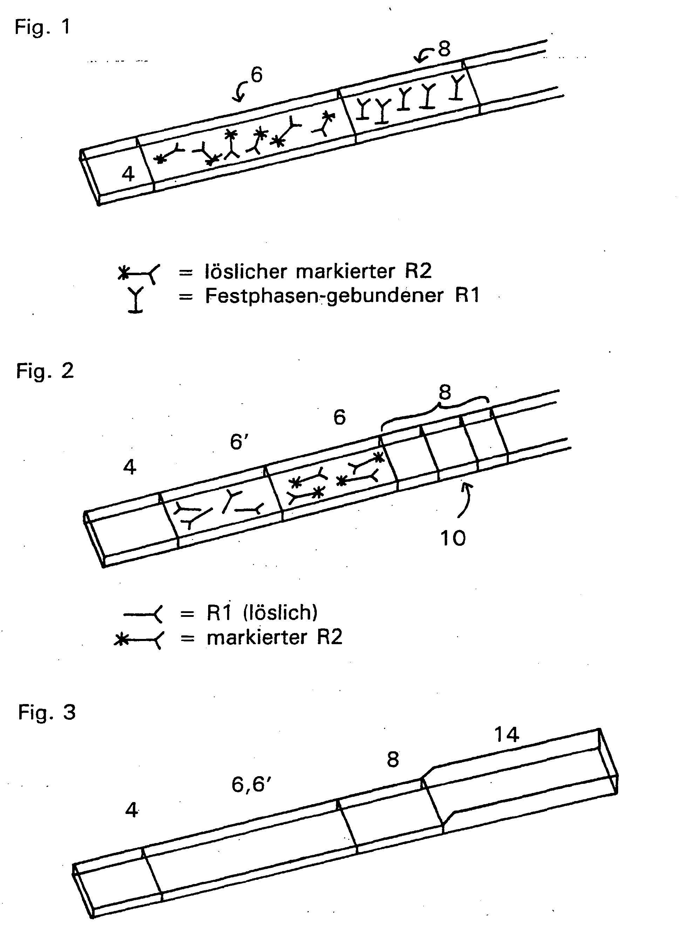

Die Figuren 1 bis 3 zeigen dabei Ausgestaltungen des erfindungsgemäßen Testkits als Teststreifen.

### Beispiel 1

Einwiegen von Stuhlproben

Ein etwa 12 ml umfassendes Einwegröhrchen und eine mikrobiologische lmpföse (z,B. Sarstedt) werden mit einer empfindlichen digitalen Laborwaage auf 0 austariert. Anschließend wird mit der Impföse der Stuhl eingewogen, indem man mit der Öse in die Stuhlprobe sticht und die an der Spitze verbleibende Stuhlmenge (etwa 100 mg) in das Einwegröhrchen einbringt. An Stelle der Impföse kann auch ein Zahnstocher benutzt werden.

Entsprechend der gewogenen Stuhlprobenmasse werden die Volumina des zuzugebenden Extraktionspuffers variiert (z.B. 100 mg Stuhl + 10 ml Puffer oder 75 mg Stuhl + 7,5 ml Puffer). End-konzentration: 10 mg Stuhl/ml Extraktionspuffer.

### Beispiel 2

Homogenisation und Extraktion der Stuhlproben

Die Stuhlprobensuspension wird bei Raumtemperatur mehrfach kräftig mit einem Reaganzglas-Schüttelgerat (z.B. VORTEX) gemixt. Die Stühle müssen gut homogenisiert sein. Um eine voll-ständige Extraktion zu gewährleisten, ist es wichtig, dem phosphatgepufferten Extraktionspuffer das Detergenz CHAPS (3-[(3-Cholamidopropyl)dimethylammoniol-1-propansulfonat, 10 mM (Sigma) beizumischen.

### Beispiel 3

### ELISA

Die verwendete ELISA-Platte ist mit einem monoklonalen Antikörper beschichtet, der nur die cytosolische NADP-abhängige Malat-Decarboxylase erkennt. Dabei bindet die cytosolische NADP-abhängige Malat-Dehydrogenase aus verdünnten Stuhlproben, aus EDTA-Plasmaproben und aus Standards an den Antikörper und wird so an der Platte immobilisiert. Ein zweiter monoklonaler Antikörper, der biotinyliert ist, bindet im nächstenn Inkubationsschritt an die cytosolische NADP-abhängige Malat-Decarboxylase. Danach bindet ein Konjugat von POD (Peroxidase) und Streptavidin an die Biotineinheit. Die Peroxidase oxidiert ABTS (2,2-Azino-bis-(3-ethylbenzo-thiazolin-6-sulfonsäure) diammoniumsalz) unter Ausbildung einer dunklen grünen Färbung. Die Konzentration von oxidiertem ABTS wird dann fotometrisch bestimmt.

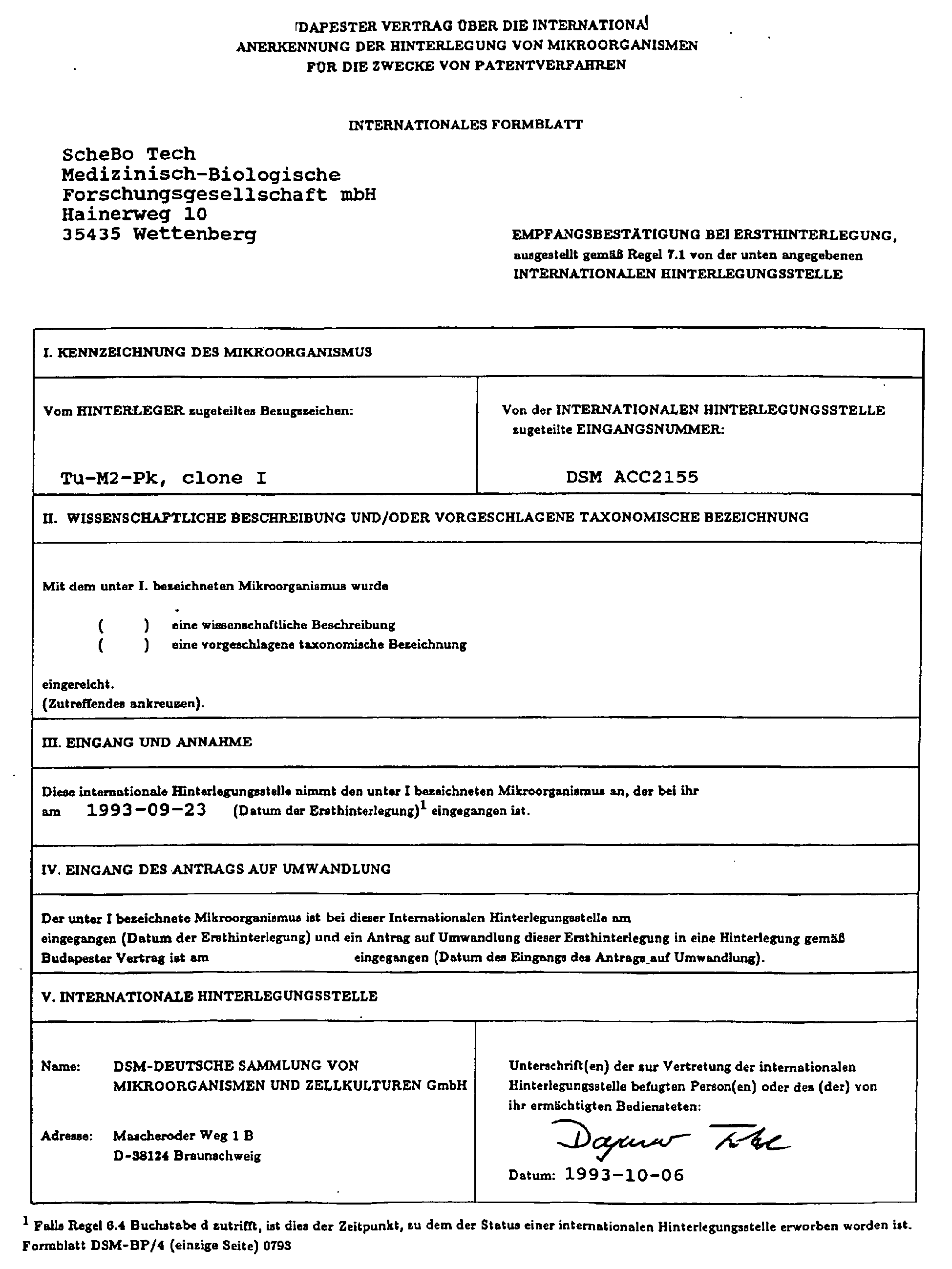
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

ScheBo Tech
Medizinisch-Biologische
Forschungsgesellschaft mbH
Hainerweg 10
35435 Wettenberg

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen: | Von der INTERNATIONALEN HINTERLEGUNGSSTELLE zugeteilte EINGANGSNUMMER:

Tu-M2-Pk, clone I | DSM ACC2155

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr
am 1993-09-23 (Datum der Ersthinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am
eingegangen (Datum der Ersthinterlegung) und ein Antrag auf Umwandlung dieser Ersthinterlegung in eine Hinterlegung gemäß
Budapester Vertrag ist am eingegangen (Datum des Eingangs des Antrags auf Umwandlung).

V. INTERNATIONALE HINTERLEGUNGSSTELLE

Name: DSM-DEUTSCHE SAMMLUNG VON
MIKROORGANISMEN UND ZELLKULTUREN GmbH

Adresse: Mascheroder Weg 1 B
D-38124 Braunschweig
| Unterschrift(en) der zur Vertretung der internationalen
Hinterlegungsstelle befugten Person(en) oder des (der) von
ihr ermächtigten Bediensteten:
Datum: 1993-10-06

¹ Falls Regel 6.4 Buchstabe d zutrifft, ist dies der Zeitpunkt, zu dem der Status einer internationalen Hinterlegungsstelle erworben worden ist.
Formblatt DSM-BP/4 (einzige Seite) 0793

## Patentansprüche

1. Verfahren zum Nachweis eines malignen Geschehens im Körper oder/und im Gastrointestinaltrakt von Menschen oder Tieren,
**dadurch gekennzeichnet,**
**dass** man in einer Körperflüssigkeitsprobe oder/und in einer Stuhlprobe das Glutaminolyse-Isoenzym cytosolische NADP-abhängige Malat-Decarboxylase oder Teilstücke derselben qualitativ oder/und quantitativ bestimmt.

2. Verfahren nach Anspruch 1,
**dadurch gekennzeichnet,**
**dass** man die Bestimmung mittels eines Immunoassays unter Verwendung mindestens eines Antikörpers, der spezifisch die cytosolische NADP-abhängige Malat-Decarboxylase oder Teilstücke derselben erkennt und mit keinem anderen Malat-Decarboxylase-Isoenzym oder Teile derselben kreuzreagiert, durchführt.

3. Verfahren nach Anspruch 2,
**dadurch gekennzeichnet,**
**dass** man als spezifischen Antikörper einen monoklonalen Antikörper verwendet.

4. Verfahren nach Anspruch 3,
**dadurch gekennzeichnet,**
**dass** man einen Antikörper verwendet, der aus der Hybridoma-Zelllinie Klon I (DSM ACC 2155) erhältlich ist, oder einen Antikörper mit gleicher Spezifität und Selektivität.

5. Verfahren nach einem der Ansprüche 1 bis 4,
**dadurch gekennzeichnet,**
**dass** man als Körperflüssigkeitsprobe Plasma, Serum, cerebrospinales Fluid, Ejakulat, Speichel oder/und Urin verwendet.

6. Verfahren nach einem der Ansprüche 2 bis 5,
**dadurch gekennzeichnet,**
**dass** man
a) die Probe mit mindestens zwei verschiedenen Rezeptoren in Kontakt bringt, von denen der erste Rezeptor R1 in fester Phase vorliegt und mit der cytosolischen NADP-abhängigen Malat-Decarboxylase bindefähig ist und der zweite Rezeptor R2 in flüssiger Phase vorliegt und ebenfalls mit der cytosolischen NADP-abhängigen Malat Decarboxylase bindefähig ist und wobei Rezeptor R2 eine Markierung trägt oder die Bindung an ein detektierbares Molekül vermittelt,
b) die feste von der flüssigen Phase trennt,
c) die Markierung oder das detektierbare Molekül in der festen Phase bestimmt und entsprechend die Menge an in der Probe vorhandener cytosolischer NADP-abhängiger Malat-Decarboxylase quantifiziert, wobei man als mindestens einen der Rezeptoren 1 oder 2 einen Antikörper verwendet, der spezifisch mit der cytosolischen NADP-abhängigen Malat-Decarboxylase bindefähig ist und kein anderes Malat-Decarboxylase-Isoenzymen bindet.

7. Verfahren nach Anspruch 6,
**dadurch gekennzeichnet,**
**dass** man den spezifisch mit der cytosolischen NADP-abhängigen Malat-Decarboxylase bindefähigen und nicht andere Malat-Decarboxylase-Isoenzyme bindenden Antikörper als Rezeptor R1 einsetzt.

8. Verfahren nach einem der Ansprüche 1 bis 7,
**dadurch gekennzeichnet,**
**dass** man als R2 einen Enzym-gebundenen Antikörper verwendet und das Verfahren als ELISA durchführt.

9. Verfahren nach Anspruch 1,
**dadurch gekennzeichnet,**
**dass** der Nachweis mit Hilfe von Massenspektroskopie, insbesondere MALDI-TOF, erfolgt.

10. Testkit zur Durchführung eines Verfahrens nach einem der Ansprüche 1 bis 9 zum Nachweis eines malignen Tumorgeschehens in Menschen oder Tieren, insbesondere im Körper oder/und im Gastrointestinaltrakt von Menschen oder Tieren,
**dadurch gekennzeichnet,**
**dass** er mindestens einen mit der cytosolischen NADP-abhängigen Malat-Decarboxylase spezifisch bindenden Rezeptor enthält, der mit keinem anderen Malat-Decarboxylase-Isoenzym kreuzreagiert, sowie gegebenenfalls weitere für die Durchführung eines Immunoassays notwendige Reagenzien, wobei der Testkit n Form eines Teststreifens (2) ausgebildet ist, der eine Auftragzone (4) für die Probe aufweist und weitere Abschnitte aufweist, welche so ausgebildet sind, dass eine im Probeauftragbereich (4) aufgetragene Flüssigkeit enthaltende Probe bis zu einer Detektionszone (8) durchwandert, in der das Testergebnis abgelesen werden kann.

11. Testkit nach Anspruch 10,
**dadurch gekennzeichnet,**
**dass** auf die Auftragzone ein Bereich (6) folgt, in dem ein löslicher markierter Antikörper vorhanden ist, der Rezeptor R2 entspricht.

12. Testkit nach einem der Ansprüche 10 oder 11,
**dadurch gekennzeichnet,**
**dass** in der Detektionszone (8) Rezeptor 1 immobilisiert vorliegt.

13. Testkit nach einem der Ansprüche 10 bis 12, **dadurch gekennzeichnet,**
**dass** zwischen der Auftragzone (4) und den weiteren Bereichen des Teststreifens ein Filter (12) angeordnet ist, der unlösliche oder gefärbte Bestandteile der Probe zurückhält.

14. Testkit nach einem der Ansprüche 10 bis 13, **dadurch gekennzeichnet,**
**dass** hinter dem Detektionsbereich (8) ein flüssigkeitsaufsaugendes Medium (14) aufgebracht ist, welches im Wesentlichen die gesamte Flüssigkeitsmenge der aufgetragenen Probe aufsaugen kann.

15. Testkit nach einem der Ansprüche 10 bis 14,
**dadurch gekennzeichnet,**
**dass** er einen weiteren Rezeptor enthält, der ebenfalls an die cytosolische NADP-abhängige Malat-Decarboxylase bindet und eine Markierung trägt oder die Bindung an ein detektierbares Molekül vermittelt.

16. Testkit nach einem der Ansprüche 10 bis 15,
**dadurch gekennzeichnet,**
**dass** er als Rezeptoren 1 oder 2 Antikörper oder spezifisch bindefähige Antikörperteile enthält.

17. Testkit nach einem der Ansprüche 10 bis 16,
**dadurch gekennzeichnet,**
**dass** Rezeptor R1 an eine feste Phase gekoppelt vorliegt.

18. Testkit nach einem der Ansprüche 10 bis 17,
**dadurch gekennzeichnet,**
**dass** er als Rezeptor 1 den monoklonalen Antikörper enthält, der aus Hybridoma-Zelllinie Klon I (DSM ACC 2155) erhältlich ist.

19. Testkit nach einem der Ansprüche 10 bis 18,
**dadurch gekennzeichnet,**
**dass** Rezeptor R2 ein löslicher markierter Antikörper ist.

20. Testkit nach einem der Ansprüche 10 bis 19,
**dadurch gekennzeichnet,**
**dass** er weitere für einen Sandwich-, ELISA-, Schwingquarz-, Mikrowaage- oder Elektrochemolumineszenztest notwendige Reagenzien oder/und Vorrichtungen enthält.

21. Verwendung eines Testkits nach einem der Ansprüche 10 bis 20 zum qualitativen oder/und quantitativen selektiven Nachweis von cytosolischer NADP-abhängiger Malat-Decarboxylase in menschlichen oder tierischen Körperflüssigkeiten oder/und in menschlichem oder tierischem Stuhl.

## Claims

1. Method for detecting a malignant process in the body or/and in the gastrointestinal tract of humans or animals,
**characterized in that**
the glutaminolysis isoenzyme cytosolic NADP-dependent malate decarboxylase or fragments thereof is/are determined qualitatively or/and quantitatively in a sample of body fluid.

2. Method according to claim 1,
**characterized in that**
the determination is carried out by means of an immunoassay using at least one antibody that specifically recognizes the cytosolic NADP-dependent malate decarboxylase or fragments thereof and does not cross-react with any other malate decarboxylase isoenzyme or parts thereof.

3. Method according to claim 2,
**characterized in that**
a monoclonal antibody is used as the specific antibody.

4. Method according to claim 3,
**characterized in that**
an antibody is used which is obtainable from the hybridoma cell line clone I (DSM ACC 2155) or an antibody is used with the same specificity and selectivity.

5. Method according to one of the claims 1 to 4,
**characterized in that**
plasma, serum, cerebrospinal fluid, ejaculate, saliva or/and urine is used as the sample of body fluid.

6. Method according to one of the claims 2 to 5,
**characterized in that**
a) the sample is contacted with at least two different receptors of which the first receptor R1 is present in a solid phase and is capable of binding to cytosolic NADP-dependent malate decarboxylase and the second receptor R2 is present in a liquid phase and is also capable of binding to cytosolic NADP-dependent malate decarboxylase, wherein receptor R2 carries a label or mediates binding to a detectable molecule,
b) the solid phase is separated from the liquid phase,
c) the label or the detectable molecule in the solid phase is determined and thus the amount of cytosolic NADP-dependent malate decarboxylase present in the sample is quantified, wherein an antibody is used as at least one of the receptors 1 or 2 which can specifically bind to cytosolic NADP-dependent malate decarboxylase and does not bind any other malate decarboxylase isoenzymes.

7. Method according to claim 6,
**characterized in that**
the antibody capable of specifically binding to cytosolic NADP-dependent malate decarboxylase and not other antibodies that bind malate decarboxylase isoenzymes is/are used as the receptor R1.

8. Method according to one of the claims 1 to 7,
**characterized in that**
an enzyme-bound antibody is used as R2 and the method is carried out as an ELISA.

9. Method according to claim 1,
**characterized in that**
the detection is carried out with the aid of mass spectroscopy, in particular MALDI-TOF.

10. Test kit for carrying out a method according to one of the claims 1 to 9 for detecting a malignant tumour process in humans or animals, especially in the body or/and in the gastrointestinal tract of humans or animals,
**characterized in that**
it contains at least one receptor that specifically binds to cytosolic NADP-dependent malate decarboxylase and cross-reacts with no other malate decarboxylase isoenzyme, and optionally other reagents necessary to carry out an immunoassay, wherein the test kit is designed in the form of a test strip (2) which has an application zone (4) for the sample and has other sections which are designed such that a sample containing liquid applied in the sample application area (4) migrates to a detection zone (8) in which the test result can be read.

11. Test kit according to claim 10,
**characterized in that**
the application zone is followed by an area (6) in which a soluble labelled antibody is present which corresponds to receptor R2.

12. Test kit according to one of the claims 10 or 11,
**characterized in that**
receptor 1 is present immobilized in the detection zone (8).

13. Test kit according to one of the claims 10 to 12,
**characterized in that**
a filter (12) which retains insoluble or coloured components of the sample is located between the application zone (4) and the other areas of the test strip.

14. Test kit according to one of the claims 10 to 13,
**characterized in that**
a liquid-absorbing medium (14) which can essentially absorb the entire amount of liquid in the applied sample is applied behind the detection area (8).

15. Test kit according to one of the claims 10 to 14,
**characterized in that**
it contains an additional receptor which also binds to cytosolic NADP-dependent malate decarboxylase and carries a label or mediates binding to a detectable molecule.

16. Test kit according to one of the claims 10 to 15,
**characterized in that**
it contains antibodies or parts of antibodies capable of specific binding as receptors 1 or 2.

17. Test kit according to one of the claims 10 to 16,
**characterized in that**
receptor R1 is present coupled to a solid phase.

18. Test kit according to one of the claims 10 to 17,
**characterized in that**
it contains the monoclonal antibody which is obtainable from the hybridoma cell line clone I (DSM ACC 2155) as receptor R1.

19. Test kit according to one of the claims 10 to 18,
**characterized in that**
receptor R2 is a soluble labelled antibody.

20. Test kit according to one of the claims 10 to 19,
**characterized in that**
it contains additional reagents or/and devices required for a sandwich, ELISA, quartz oscillator, microbalance or electrochemiluminescence test.

21. Use of a test kit according to one of the claims 10 to 20 for the qualitative or/and quantitative selective detection of cytosolic NADP-dependent malate decarboxylase in human or animal body fluids or/and in human or animal faeces.

## Revendications

1. Procédé de détection d'un processus malin dans le corps et/ou le tractus gastro-intestinal d'un homme ou d'un animal, **caractérisé en ce que** l'on déterminé de manière qualitative ou quantitative, dans un échantillon de fluide corporel et/ou un échantillon de selles, l'isoenzyme de glutaminolyse malate décarboxylase cytosolique, NADP-dépendante, de la glutaminolyse ou des fragments de celui-ci.

2. Procédé selon la revendication 1, **caractérisé en ce que** l'on réalise la détermination à l'aide d'un immunodosage en utilisant au moins un anticorps, qui reconnaît spécifiquement la malate décarboxylase cytosolique, NADP-dépendante, ou des fragments de celle-ci et qui ne réagit de manière croisée avec aucune autre isoenzyme de malate décarboxylase ou fragments de celle-ci.

3. Procédé selon la revendication 2, **caractérisé en ce que** l'on utilise comme anticorps spécifique, un anticorps monoclonal.

4. Procédé selon la revendication 3, **caractérisé en ce que** l'on utilise un anticorps, qui est obtenu à partir de la lignée cellulaire clone I d'hybridome (DSM ACC 2155) ou un anticorps avec les mêmes spécificité et sélectivité.

5. Procédé selon l'une quelconque des revendications 1 à 4, **caractérisé en ce que** l'on utilise comme échantillon de fluide corporel, le plasma, le sérum, le fluide cérébrospinal, le sperme, la salive et/ou l'urine.

6. Procédé selon l'une quelconque des revendications 2 à 5, **caractérisé en ce que** l'on
a) met en contact l'échantillon avec au moins deux récepteurs différents, dont le premier récepteur R1 est en phase solide et peut se lier à la malate décarboxylase cytosolique, NADP-dépendante, et le deuxième récepteur R2 est présent en phase liquide et peut également se lier à la malate décarboxylase cytosolique, NADP-dépendante, et où le récepteur R2 porte un marquage ou permet la liaison à une molécule détectable,
b) sépare la phase solide de la phase liquide,
c) détermine le marquage ou la molécule détectable dans la phase solide et de manière correspondante, on quantifie la quantité de malate décarboxylase cytosolique, NADP-dépendante, présente dans l'échantillon, où on utilise comme au moins un des récepteurs 1 ou 2, un anticorps, qui peut se lier de manière spécifique à la malate décarboxylase cytosolique, NADP-dépendante, et à aucune autre isoenzyme de malate décarboxylate.

7. Procédé selon la revendication 6, **caractérisé en ce que** l'on met en oeuvre comme récepteur R1, l'anticorps, qui peut se lier spécifiquement à la malate décarboxylase cytosolique, NADP-dépendante, et ne liant pas d'autre isoenzyme de malate décarboxylase.

8. Procédé selon l'une quelconque des revendications 1 à 7, **caractérisé en ce que** l'on utilise comme R2, un anticorps lié à une enzyme et le procédé est réalisé comme une ELISA.

9. Procédé selon la revendication 1, **caractérisé en ce que** l'on réalise la détection à l'aide de la spectrométrie de masse, en particulier le MALDI-TOF.

10. Kit de test pour réaliser un procédé selon l'une quelconque des revendications 1 à 9, pour la détection d'un processus tumoral malin chez l'homme ou l'animal, en particulier dans le corps et/ou le tractus gastro-intestinal d'un homme ou d'un animal, **caractérisé en ce qu'**il contient au moins un récepteur se liant spécifiquement à la malate décarboxylase cytosolique, NADP-dépendante, lequel ne réagit de manière croisée, avec aucune autre isoenzyme de malate décarboxylase, ainsi que le cas échéant, d'autres réactifs nécessaires à la réalisation d'un immunodosage, où le kit de test a la forme d'une bande de test (2), qui présente une zone d'application (4) de l'échantillon et d'autres segments, qui sont formés de sorte qu'un échantillon contenant un liquide appliqué sur la zone d'application de l'échantillon (4) migre jusqu'à une zone de détection (8), dans laquelle on peut lire le résultat du test.

11. Kit de test selon la revendication 10, **caractérisé en ce qu'**après, la zone d'application, suit un domaine (6), dans lequel est présent un anticorps soluble marqué, qui correspond au récepteur R2.

12. Kit de test selon l'une quelconque des revendications 10 ou 11, **caractérisé en ce que** dans la zone de détection (8), est présent le récepteur 1 immobilisé.

13. Kit de test selon l'une quelconque des revendications 10 à 12, **caractérisé en ce qu'**entre la zone d'application (4) et les autres domaines de la bande de test, est disposé un filtre (12), qui retient les constituants insolubles ou colorés de l'échantillon.

14. Kit de test selon l'une quelconque des revendications 10 à 13, **caractérisé en ce qu'**après la zone de détection (8), est appliqué un milieu absorbant les liquides (14), qui peut absorber essentiellement, toute la quantité de liquide de l'échantillon appliqué.

15. Kit de test selon l'une quelconque des revendications 10 à 14, **caractérisé en ce qu'**il contient un autre récepteur, qui lie également la malate décarboxylase cytosolique, NADP-dépendante, et qui porte un marquage ou permet la liaison à une molécule détectable.

16. Kit de test selon l'une quelconque des revendications 10 à 15, **caractérisé en ce qu'**il contient comme récepteurs 1 ou 2, des anticorps ou des parties d'anticorps se liant spécifiquement.

17. Kit de test selon l'une quelconque des revendications 10 à 16, **caractérisé en ce que** le récepteur R 1 est couplé à une phase solide.

18. Kit de test selon l'une quelconque des revendications 10 à 17, **caractérisé en ce qu'**il contient comme récepteur 1, l'anticorps monoclonal qui est obtenu à partir de la ligné cellulaire clone I d'hybridome (DSM ACC 2155)

19. Kit de test selon l'une quelconque des revendications 10 à 18, **caractérisé en ce que** le récepteur R2 est un anticorps marqué soluble.

20. Kit de test selon l'une quelconque des revendications 10 à 19, **caractérisé en ce qu'**il contient les réactifs et/ou dispositifs nécessaires à un test en sandwiche, ELISA, à quartz oscillant, à micro-ondes ou à électrochimioluminescence.

21. Utilisation d'un kit de test selon l'une quelconque des revendications 10 à 20, pour la détection sélective qualitative et/ou quantitative de la malate décarboxylase cytosolique, NADP-dépendante, dans des fluides corporels humains ou animaux ou/et dans les selles humaines ou animales.
